# EUROPÄISCHE PATENTSCHRIFT

(11) **EP 0 597 999 B1**
(45) Veröffentlichungstag und Bekanntmachung des Hinweises auf die Patenterteilung: **06.11.1996**
(21) Anmeldenummer: 92917182.5
(22) Anmeldetag: 01.08.1992
(51) Int. Cl.: C09J 7/02, C09J 7/04, A61L 15/58

(54) **HAFTKLEBEBAND**
PRESSURE-SENSITIVE ADHESIVE STRIP
BANDE AUTO-ADHESIVE

(30) Priorität: 08.08.1991 DE 4126230
(43) Veröffentlichungstag der Anmeldung: 25.05.1994
(73) Patentinhaber: Lohmann GmbH & Co. KG, 56567 Neuwied (DE)
(72) Erfinder: CZECH, Zbigniew, D-5400 Koblenz 1 (DE); NISSING, Peter, D-4010 Hilden (DE); MILKER, Roland, D-5457 Oberhonnefeld (DE); WEHMANN, Jörg, D-5450 Neuwied 1 (DE)
(74) Vertreter: Flaccus, Rolf-Dieter, Dr.
(86) Internationale Anmeldenummer: EP9201749
(87) Internationale Veröffentlichungsnummer: WO9303106

(56) Entgegenhaltungen:
- EP-A- 0 100 146
- EP-A- 0 118 726
- US-A- 4 413 080

## Beschreibung

Die Erfindung betrifft ein sterilisierbares Haftklebeband mit einer wasserlöslichen Haftklebemasse und einem wasserlöslichen oder wasserdispergierbaren Träger.

Klebebänder der genannten Art sind prinzipiell als sog. redispergierbare ein- und doppelseitige Spleißbänder bekannt.
Wasserlöslich im eigentlichen Sinne ist nur die Haftklebemasse, während das Trägermaterial bei mechanischer Einwirkung zerfasert (redispergiert) wird.
Die Wasserlöslichkeit des Haftklebstoffes beruht auf der Hydrophilie der in der Polymerkette eingebauten Segmente. Durch eine Kombination von verschiedenen mehr oder weniger wasserlöslichen Komponenten wird die vollständige Löslichkeit in Wasser sowohl im alkalischen bis neutralen wie auch im sauren pH-Bereich erzielt.
Eine Zugabe von wasserlöslichen Alkanolaminen erniedrigt die Glasumwandlungstemperatur des relativ harten Polymerisates und verleiht ihm eine sehr gute Anfaßklebkraft.
Zwecks Erhöhung der Wasserlöslichkeit wird oft eine Neutralisation der Carboxylgruppen mittels Alkalien vorgenommen. Um die Anfaßklebrigkeit (Tack) zu steigern, können außerdem klebrigmachende Harze oder wasserlösliche Weichmacher zugesetzt werden.

Eine Fülle von Patentliteratur spiegelt dies beim Stand der Technik wieder und zeigt auf, welche Wege insbesondere in Richtung Wasserlöslichkeit von Haftklebern bisher beschritten wurden. Hierfür typisch sind die folgenden Beispiele:
Carboxylgruppenhaltige Copolymerisate auf Isooctylacrylat/Butylacrylat-Basis in Mischung mit wasserlöslichen Polyoxyethylenderivaten und Tallharztackifiern (US-A 4 413 080). Derartige Copolymerisate zeichnen sich aus durch eine hervorragende Wasserlöslichkeit. Bei der Sterilisation bilden sich jedoch Abbauprodukte, was die physiologische Unbedenklichkeit gefährdet.

Wasserlösliche Haftklebemassen auf Basis von 2-Ethylhexylacrylat, Methacrylsäure und hydroxylgruppenhaltigen (Meth)acrylaten (EP-B 0 147 067). Diese Haftklebemassen besitzen ausgezeichnete Wasserlöslichkeit und lassen sich problemlos sterilisieren. Sie weisen jedoch eine mangelhafte Resistenz gegenüber Hautfeuchte auf, die ihre Einsatzmöglichkeiten stark einschränkt.

Wasserlösliche Haftklebemassen aufgebaut aus Vinylphosponsäure, Acryl- oder Methacrylamid, Vinylsulphonsäure und Vinylphosphonsäureestern (US-A 4 518 745). Sie sind beständig gegen Hautfeuchte, lassen sich einwandfrei sterilisieren und besitzen gute Wasserlöslichkeit. Aufgrund der relativ hohen Konzentration an Restmonomeren ist die Hautverträglichkeit und die physiologische Unbedenklichkeit in Frage gestellt.

Wasserlösliche Haftkleber auf Polyvinylcarbonsäurebasis mit mindestens einem hydroxylgruppenhaltigen Weichmacher (Molekularmasse < 1000), die ggf. mit einem Vernetzer vernetzt sind. Dieses Klebematerial enthält als Hauptbestandteile ein Copolymerisat aus β-Acryloyloxypropionsäure mit anderen ungesättigten Verbindungen (z.B. Methacrylsäure) und ein Polyoxyalkylen sowie ggf. weitere Komponenten wie Vernetzer, Lösemittel, Färbemittel, Füllstoffe und Alterungsinhibitoren (DE 38 25 527).

Wasserlösliches Copolymerisat aus Vinylcarbonsäure, hydroxylgruppenhaltigem (Meth)acrylat, (Meth)acrylamid-Derivat und wenigstens einem Vinylcarbonsäuresalz. Die Masse wird mit einem wasserlöslichen Weichmacher beispielsweise aus der Gruppe der Polyoxyalkylene modifiziert (DE 3901 690).

Die dem Stand der Technik gemäß DE 38 25 527 und DE 39 01 690 entsprechenden "redispergierbaren" Haftklebebänder eignen sich beispielsweise bevorzugt für die Anlängung von Papierbahnen, weisen aber entscheidende Nachteile bei Anwendungen im Hygienebereich auf, worunter die temporäre Fixierung von textilen Flächengebilden auf der menschlichen Haut verstanden wird. So ist eine gleich hohe spezifische Haftung auf der Haut wie auch auf den textilen Flächengebilden nicht gegeben. Besonders schwach ausgeprägt ist die Resistenz gegenüber der Hautfeuchte, die normalerweise zu einer Erweichung der wasserlöslichen Klebmasse bis hin zum Kohäsionsbruch führt. Die Rezeptierung üblicher wasserlöslicher Klebmassen berücksichtigte bislang auch nicht die beispielsweise vom Bundesgesundheitsamt vorgeschriebenen Waschbedingungen, die sich ganz erheblich von den Verhältnissen in einem Papierpulper unterscheiden.
Dies betrifft insbesondere textile Flächengebilde wie beispielsweise Abdecktücher für Operationen.
Besonders nachteilig fällt ins Gewicht, daß der Klebebandträger lediglich redispergierbar und nicht vollständig wasserlöslich ist, wodurch es zu einer Kontamination des gewaschenen textilen Flächengebildes mit Fasern kommt, die aus dem Klebebandträger stammen.

Der vorliegenden Erfindung lag somit die Aufgabe zugrunde, ein Haftklebeband der eingangs genannten Art für den speziellen Einsatz im Hygienebereich zu schaffen, welches frei von den oben geschilderten Mängeln ist. Insbesondere soll das Haftklebeband hautverträglich, physiologisch unbedenklich und problemlos sterilisierbar sein sowie Resistenz gegenüber der Hautfeuchte aufweisen. Diese Aufgabe wird erfindungsgemäß dadurch gelöst, daß die verwendete Haftklebemasse folgende Komponenten enthält.
a. 40-65 Gew.-% eines carboxylgruppenhaltigen Polymerisats auf Acrylatbasis
   wobei das carboxylgruppenhaltige Polymerisat auf Acrylatbasis
   a 1. 50-80 Gew.-% Alkyl(meth)acrylate mit 4-12 C-Atomen im Alkylrest,
   a 2. mindestens 10 Gew.-% einfach ungesättigte Carbonsäuren
   a 3. 1-20 Gew.-% Vinyl-Phosphonsäure enthält,
b. 30-55 Gew.-% eines wasserlöslichen Amins, insbesondere Alkanolamin
c. 3-11 Gew.-% eines weichmachenden Harzes,
   wobei das weichmachende Harz ein mit Methanol, Triethylenglykol oder Glycerin verestertes hydriertes Kolophoniumharz ist,
d. 0,5 - 3 Gew.-% carboxylgruppenhaltige , nicht auf Acrylat basierende Polymere,
   wobei diese aus Alkylenglykol und Dicarbonsäure herge-stellte Polyester oder auf Polyisoprenbasis aufgebaut sind,
e. 0,05 Gew.-% eines Vernetzungsmittels.

Das Alkyl (Meth)acrylat mit 4 bis 12 C-Atomen ist vorteilhaft ein Butyl-, Pentyl-, Hexyl-, Heptyl-, Octyl-, 2-Ethylhexyl-, Isooctyl-, 2-Methylheptyl-, Nonyl-, Decyl- oder Dodecylmethacrylat.

Als bevorzugte einfach ungesättigte Carbonsäuren können (Meth)acrylsäure, β-Acryloyloxypropionsäure, Vinylessigsäure, Fumarsäure, Crotonsäure, Aconitsäure, Dimethylacrylsäure oder Itaconsäure verwendet werden.

Im Falle des wasserlöslichen Amins wird bevorzugt ein Alkanolamin wie Monoethanolamin, Diethanolamin, Triethanolamin, Dimethylaminoethanol, Diethylaminoethanol, N-Methylethanolamin, Monoisopropanolamin, Diisopropanolamin, Triisopropanolamin eingesetzt.

Das die Klebemasse weichmachende Harz ist ein hydrierter Kolophoniumharzester mit Methanol, Triethylenglykol oder Glycerin als Alkoholkomponente.

Die carboxylgruppenhaltigen, nicht auf Acrylat basierenden Polymere sind Polyester, die durch Polykondensation von Dicarbonsäuren oder ihren funktionellen Derivaten wie Anhydriden und Dialkoholen wie z.B. Ethylenglykol hergestellt wurden oder Polymere auf Polyisoprenbasis.

Als Vernetzungsmittel können vorteilhaft Metallchelate, Metallsäureester, Epoxyd-, Aziridin- oder Melaminformaldehydharze zur Verbesserung der thermischen Belastbarkeit der wasserlöslichen Klebemasse eingesetzt werden.

Als "Rückgrat"-Polymer für die Haftklebemasse wird bevorzugt ein Polyacrylat verwendet, dessen Wasserlöslichkeit im alkalischen Bereich (pH größer 8) und bei erhöhter Temperatur (oberhalb 60°C) überproportional ansteigt. Eine Redispergierbarkeit des Klebebandträgers wird vorzugsweise durch Verwendung von dünnen, leicht zerfaserbaren Papieren (z.B. Teefilterpapier, Zigarettenpapier) erzielt.

Eine gute Wasserlöslichkeit stellt sich ein, wenn als Trägermaterial ein Vliesstoff verwendet wird, der wasserlösliche Fasern wie beispielsweise Calciumalginatfasern oder Polyvinylalkoholfasern enthält. Eine weitere vorteilhafte Ausführungsform der Erfindung verwendet wasserlösliche Polyvinylalkohol-, Gelatine- oder Gelatine/Glycerin-Folien als Träger.

Das erfindungsgemäße Haftklebeband läßt sich vorteilhaft überall da einsetzen, wo neben einer vollständigen Wasserlöslichkeit eine hohe Hautverträglichkeit, gepaart mit einer hohen spezifischen Haftung auf den unterschiedlichsten textilen Flächengebilden gefordert wird, wie beispielsweise auf Baumwollmischgeweben, Baumwollgeweben, nicht imprägniert oder mit Fluorcarbonharz imprägniert, Mikrofilamentgeweben oder auf Laminaten aus Polyesterfasern/-PTFE-Membranen.

Ein bevorzugtes Anwendungsgebiet ist beispielsweise die temporäre Fixierung von Operationsabdecktüchern auf der menschlichen Haut, da die mit der Erfindung erzielten Vorteile insbesondere darin bestehen, daß neben der reinen Haftklebefunktion das Merkmal einer vollständigen Wasserlöslichkeit unter den für solche Abdecktücher typischen Waschbedingungen besteht.

Dies wird nicht unter Verzicht auf sonstige wichtige Eigenschaften wie Hautverträglichkeit oder Sterilisierbarkeit erreicht.

Das erfindungsgemäße Haftklebeband trägt darüber hinaus vorteilhaft zu einer Verminderung des Anteils an Einwegmaterialien im Operationsbereich bei, deren Entsorgung umweltbelastend, zeitaufwendig und teuer ist. Mit dem Haftklebeband nach der Erfindung wird somit die Ökobilanz von Operationsabdeckmaterialien entscheidend verbessert.

Ein weiteres bevorzugtes Anwendungsgebiet ist der Windelverschluß, wobei die Wiederverwertung von Baumwollwindeln durch das erfindungsgemäße Haftklebeband wesentlich gefördert wird.

Ausführungsbeispiele der Erfindungen werden im folgenden näher beschrieben:

Das carboxylgruppenhaltige Acrylatcopolymer wird in üblicher Weise durch radikalische Polymerisation in einem organischen Lösungsmittel hergestellt. Als Lösungsmittel empfiehlt sich je nach der Monomerenzusammensetzung Aceton oder ein Gemisch Aceton/2-Propanol im Verhältnis 1:1. Die Polymerisation wird mit einem Feststoffgehalt von 50 Gew.-% durchgeführt, das erhaltene Polymerisat wird mit einem wasserlöslichen Amin, einem weichmachenden Harz, einem carboxylgruppenhaltigen, nicht auf Acrylat basierenden Polymer und einem Vernetzungsmittel gemischt.

Die nachfolgende Tabelle 1 enthält eine Zusammenstellung möglicher Polymerisationszusammensetzung, aus Tabelle 2 sind Zusammensetzungen für die Haftklebemasse des erfindungsgemäßen Haftklebebandes zusammengestellt.

Die dabei verwendeten Abkürzungen haben folgende Bedeutung:

### Abkürzungsverzeichnis

- 2-EHA: - 2-Ethylhexylacrylat
- BA: - Butylacrylat
- AS: - Acrylsäure
- MAS: - Methacrylsäure
- VPS: - Vinylphosphonsäure
- TA: - Triethanolamin
- DIA: - Diisopropanolamin
- C: - Weichharz Cellolyn 21®
- HD: - Weichharz Hercolyn D®
- CPE: - carboxylgruppenhaltiger Polyester
- LIR 410: - carboxylgruppenhaltiges Polyisopren
- ZrACA: - Zirkonacetylacetonat
- MFH: - Melaminformaldehydharz

**Tabelle 1**

| Beispiel | Polymerisationszusammensetzung [Gew.-%] | | | | |
|---|---|---|---|---|---|
| | C₄-C₁₂ Alkyl(meth)acryl | | Vinylcarbonsäure | | VPS |
| | 2-EHA | BA | AS | MAS | |
| 1 | 40 | 40 | 15 | -- | 5 |
| 2 | 60 | 10 | 5 | 20 | 5 |
| 3 | -- | 70 | 5 | 5 | 20 |
| 4 | 60 | -- | 20 | 18 | 2 |
| 5 | 10 | 70 | 17 | -- | 3 |
| 6 | 60 | 20 | 16 | -- | 4 |
| 7 | 60 | -- | 35 | -- | 5 |
| 8 | -- | 50 | 40 | -- | 10 |
| 9 | 35 | 35 | 15 | 10 | 5 |
| 10 | -- | 80 | -- | 19 | 1 |

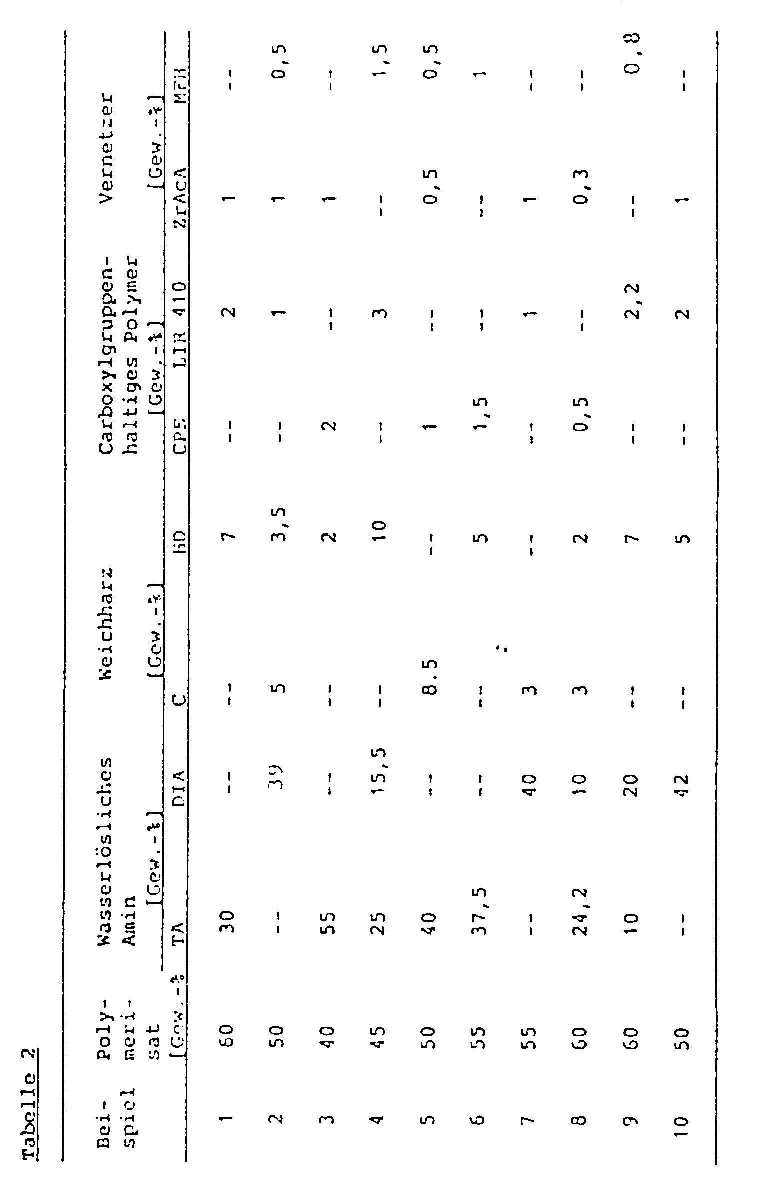

Die Haftklebemassen werden mittels eines Walzenrakels auf eine siliconisierte Polyesterfolie aufgetragen und im Trockenkanal 10 Minuten bei 100°C getrocknet. Nach dem Trocknen werden die Haftklebefilme beidseitig auf eine Polyvinylalkohol-Trägerfolie kaschiert. Die auf diesem Weg hergestellten doppelseitigen Klebebänder werden anschließend einem Löslichkeitstest unterzogen.
Zur Prüfung der Wasserlöslichkeit werden 250 ml destilliertes Wasser mit Natriumhydroxid auf pH 9 eingestellt und auf 65°C mittels eines Heizrührmotors erhitzt. Ist die Temperatur erreicht, werden 6,25 cm² Klebeband bei 400 U/min gelöst. Die kompletten Klebebandsysteme lösten sich innerhalb von 10 min vollständig auf.

Die Ermittlung der primären Verträglichkeit der so hergestellten Klebebänder an der intakten und skarifizierten blanken Haut von Kaninchen ergab gemäß OECD-Guidelines Nr. 404 die Einstufung "nicht hautreizend" (mittlerer Irritationsindex 0,0).

Auch die biologische Abbaubarkeit der wasserlöslichen Haftklebemassen ist gegeben, wie analytische Arbeiten in Anlehnung an die in den standardisierten DIN-Normen niedergelegten Verfahren aufzeigen. Danach ist die biochemische Oxidation innerhalb von 12 Tagen zu etwa 75% abgeschlossen.

## Patentansprüche

1. Sterilisierbares Haftklebeband mit einer wasserlöslichen Haftklebemasse und einem wasserlöslichen oder wasserdispergierbaren Träger, dadurch gekennzeichnet, daß die Haftklebemasse folgende Komponenten enthält:
a. 40-65 Gew.-% eines carboxylgruppenhaltigen Polymerisats auf Acrylatbasis,
wobei das carboxylgruppenhaltige Polymerisat auf Acrylatbasis
a 1. 50-80 Gew.-% Alkyl(meth)acrylate mit 4-12 C-Atomen im Alkylrest,
a 2. mindestens 10 Gew.-% einfach ungesättigte Carbonsäuren
a 3. 1-20 Gew.-% Vinyl-Phosphonsäure enthält,
b. 30-55 Gew.-% eines wasserlöslichen Amins, insbesondere Alkanolamin
c. 3-11 Gew.-% eines weichmachenden Harzes,
wobei das weichmachende Harz ein mit Methanol, Triethylenglykol oder Glycerin verestertes hydriertes Kolophoniumharz ist,
d. 0,5-3 Gew.-% carboxylgruppenhaltige, nicht auf Acrylat basierende Polymere,
wobei diese aus Alkylenglykol und Dicarbonsäurehergestellte Polyester oder Polyisoprenbasis aufgebaut sind,
e. 0,05-1,5 Gew.-% eines Vernetzungsmittels.

2. Haftklebeband nach Anspruch 1, dadurch gekennzeichnet, daß das Alkyl (meth)acrylat mit 4-12 C-Atomen im Alkylrest ein Butyl-, Pentyl-, Hexyl-, Octyl-,2-Ethylhexyl, Isooctyl-, 2-Methylheptyl-, Nonyl-, Decyl- oder Dodecyl(meth)acrylat ist.

3. Haftklebeband nach Anspruch 1 , dadurch gekennzeichnet,daß die einfach ungesättigte Carbonsäure (Meth)-acrylsäure β-Acryloyloxypropionsäure, Vinylessigsäure, Fumarsäure, Crotonsäure, Aconitsäure, Dimethylacrylsäure oder Itaconsäure ist.

4. Haftklebeband nach Anspruch 1, dadurch gekennzeichnet, daß das wasserlösliche Amin ein Alkanolamin aus der Gruppe Monoethanolamin, Diethanolamin, Triethanolamin, Dimethylaminoethanol, Diethylaminoethanol, N-Methylethanolamin, Monoisopropanolamin, Diisopropanolamin, Triisopropanolamin ist.

5. Haftklebeband nach Anspruch 1, dadurch gekennzeichnet, daß das Vernetzungsmittel aus der Gruppe der Metallchelate, Metallsäureester, Epoxid-, Aziridin- oder Melaminformaldehydharze ausgewählt ist.

6. Haftklebeband nach einem oder mehreren der vorangehenden Ansprüche,dadurch gekennzeichnet, daß als "Rückgrat-Polymer" für die Haftklebemasse bevorzugt eine Polyacrylat verwendet ist, dessen Wasserlöslichkeit im alkalischen Bereich (pH>8) und bei erhöhter Temperatur >60° C überproportional ansteigt.

7. Haftklebeband nach einem oder mehreren der vorangehenden Ansprüche, dadurch gekennzeichnet, daß die Haftklebemasse nicht hautreizend ist.

8. Haftklebeband nach einem oder mehreren der vorangehenden Ansprüche, dadurch gekennzeichnet, daß die wasserlösliche Haftklebemasse biologisch abbaubar ist.

9. Haftklebeband nach einem oder mehreren der vorangehenden Ansprüche, dadurch gekennzeichnet, daß als Klebebandträger Papiere, Vliesstoffe oder Folien eingesetzt werden, die in Wasser redispergierbar oder wasserlöslich sind.

10. Haftklebeband nach Anspruch 9, dadurch gekennzeichnet, daß der Vliesstoff Calciumalginatfasern oder Polyvinylalkoholfasern enthält.

11. Haftklebeband nach Anspruch 9, dadurch gekennzeichnet, daß der Klebebandträger eine Polyvinylalkohol-, Gelatine- oder eine Gelatine/Glycerin-Folie ist.

12. Verwendung des Haftklebebandes nach einem oder mehreren der vorangehenden Ansprüche für die temporäre Fixierung von textilen Flächengebilden auf der menschlichen Haut und zum Windelverschluß.

## Claims

1. Sterilizable pressure-sensitive adhesive tape comprising a water-soluble pressure-sensitive adhesive mass and a water-soluble or water-dispersible carrier, characterized in that said pressure-sensitive adhesive mass contains the following components:
a. 40 - 65%-wt. of a carboxyl group-containing poly merisate based on acrylate,
said carboxyl group-containing acrylate-based polymerisate containing
a 1. 50 - 80%-wt. alkyl(meth)acrylate with 4 - 12 C-atoms in the alkyl residue,
a 2. at least 10%-wt. unsaturated carboxylic acids,
a 3. 1 - 20%-wt. vinyl-phosponic acid;
b. 30 - 55%-wt. of a water-soluble amine, in particular alkanolamine;
c. 3 - 11%-wt of a plasticizing resin,
said plasticizing resin being a colophony resin esterified with methanol, triethylene glykol or glycerol;
d. 0.5 - 3%-wt. carboxyl group-containing, non-acrylate-based polymers,
said non-acrylate-based polymers being built up of alkylene glykol and dicarboxylic acid, or on a polyisoprene basis,
e. 0.05 - 1.5%-wt. of a crosslinking agent.

2. The pressure-sensitive adhesive tape according to claim 1, characterized in that the alkyl(meth)acrylate with 4 - 12 C-atoms in the alkyl residue is a butyl, pentyl, hexyl, octyl, 2-ethylhexyl, isooctyl, 2-methylheptyl, nonyl, decyl or dodecyl(meth)acrylate.

3. The pressure-sensitive adhesive tape according to claim 1, characterized in that the monounsaturated carboxylic acid is a (meth)acrylic acid, β-acryloyloxypropionic acid, vinylacetic acid, fumaric acid, crotonic acid, aconitic acid, dimethylacrylic acid or itaconic acid.

4. The pressure-sensitive adhesive tape according to claim 1, characterized in that the water-soluble amine is an alkanolamine of the group of monoethanolamine, diethanolamine, triethanolamine, dimethylaminoethanol, diethylaminoethanol, n-methylethanolamine, monoisopropanolamine, diisopropanolamine, triisopropanolamine.

5. The pressure-sensitive adhesive tape according to claim 1, characterized in that the cross-linking agent is selected from the group of metal chelates, metal acid esters, epoxide, aziridine or melamine formaldehyde resins.

6. The pressure-sensitive adhesive tape according to one or more of the preceding claims, characterized in that as "backbone polymer" for the pressure-sensitive adhesive mass a polyacrylate is preferably used, the water solubility of which is in the alkaline range (pH > 8) and increases superproportionally at elevated temperature >60°C.

7. The pressure-sensitive adhesive tape according to one or more of the preceding claims, characterized in that the pressure-sensitive adhesive mass is non-skin-irritating.

8. The pressure-sensitive adhesive tape accordidng to one or more of the preceding claims, characterized in that the water-soluble pressure-sensitive adhesive mass is biologically degradable.

9. The pressure-sensitive adhesive tape according to one or more of the preceding claims, characterized in that as adhesive tape carriers papers, nonwovens, films or sheets are used which are redispersible in water or water-soluble.

10. The pressure-sensitive adhesive tape according to claim 9, characterized in that the nonwoven contains calcium alginate fibres or polyvinyl alcohol fibres.

11. The pressure-sensitive adhesive tape according to claim 9, characterized in that the adhesive tape carrier is a polyvinyl alcohol film, gelatin film or gelatin/glycerol film.

12. The use of the pressure-sensitive adhesive tape according to one or more of the preceding claims for the temporary fixation of textile fabrics on the human skin and for fastening diapers.

## Revendications

1. Bande autocollante stérilisable pourvue d'une masse de colle de contact soluble dans l'eau et d'un support soluble dans l'eau ou dispersible dans l'eau, caractérisée en ce que la masse de colle de contact contient les composants qui suivent :
a. 40 à 65% en poids d'un polymère à base d'acrylate contenant des radicaux carboxyle,
où le polymère à base d'acrylate contenant des radicaux carboxyle contient
a 1. 50 à 80% en poids de (méth)acrylates d'alkyle dont le radical alkyle comporte de 4 à 12 atomes de carbone,
a 2. au moins 10% en poids d'acides carboxyliques monoinsaturés,
a 3. 1 à 20% en poids d'acide vinylphosphonique,
b. 30 à 55% en poids d'une amine soluble dans l'eau, plus particulièrement une alcanolamine,
c. 3 à 11% en poids d'une résine plastifiante, où la résine plastifiante est une résine de colophane hydrogénée, estérifiée avec du méthanol, du triéthylèneglycol ou de la glycérine,
d. 0,5 à 3% en poids de polymères qui ne sont pas à base d'acrylate, contenant des radicaux carboxyle,
où ceux-ci sont à base de polyisoprène ou de polyesters préparés à partir d'alkylèneglycols et d'acides dicarboxyliques,
e. 0,05 à 1,5% en poids d'un agent de réticulation.

2. Bande autocollante suivant la revendication 1, caractérisée en ce que le (méth)acrylate d'alkyle avec un radical alkyle comportant de 4 à 12 atomes de carbone est du (méth)acrylate de butyle, de pentyle, d'hexyle, d'octyle, de 2-éthylhexyle, d'isooctyle, de 2-méthylheptyle, de nonyle, de décyle, de dodécyle.

3. Bande autocollante suivant la revendication 1, caractérisée en ce que l'acide carboxylique monoinsaturé est l'acide (méth)acrylique, l'acide β-acryloyloxypropionique, l'acide vinylacétique, l'acide fumarique, l'acide crotonique, l'acide aconitique, l'acide diméthylacrylique, ou l'acide itaconique.

4. Bande autocollante suivant la revendication 1, caractérisée en ce que l'amine soluble dans l'eau est une alcanolamine appartenant au groupe formé par la monoéthanolamine, la diéthanolamine, la triéthanolamine, le diméthylaminoéthanol, le diéthylaminoéthanol, la N-méthyléthanolamine, lamonoisopropanolamine, la diisopropanolamine et la triisopropanolamine.

5. Bande autocollante suivant la revendication 1, caractérisée en ce que l'agent de réticulation est choisi dans le groupe formé par les chélates de métaux, les esters d'acides de métaux, les résines époxydes, d'aziridine, ou de mélamine-formaldéhyde.

6. Bande autocollante suivant l'une ou plusieurs des revendications précédentes, caractérisée en ce qu'à titre de "polymère d'ossature" pour la masse de colle de contact, on utilise, de préférence, un polyacrylate dont l'hydrosolubilité s'élève de manière superproportionnelle dans la plage alcaline (pH>8) et à température élevée supérieure à 60°C.

7. Bande autocollante suivant l'une ou plusieurs des revendications précédentes, caractérisée en ce que la masse de colle de contact n'est pas irritante vis-à-vis de la peau.

8. Bande autocollante suivant l'une ou plusieurs des revendications précédentes, caractérisée en ce que la masse de colle de contact soluble dans l'eau est biologiquement dégradable.

9. Bande autocollante suivant l'une ou plusieurs des revendications précédentes, caractérisée en ce qu'à titre de supports de la bande autocollante, on utilise des papiers, des nappes ou mats, ou des feuilles, qui sont redispersibles dans l'eau, ou solubles dans l'eau.

10. Bande autocollante suivant la revendication 9, caractérisée en ce que la nappe ou le mat contient des fibres d'alginate calcique, ou des fibres de poly(alcool vinylique).

11. Bande autocollante suivant la revendication 9, caractérisée en ce que le support de la bande autocollante est une feuille de poly(alcool vinylique), de gélatine, ou de gélatine/glycérine.

12. Utilisation de la bande autocollante suivant une ou plusieurs des revendications précédentes, en vue de la fixation temporaire d'articles plats textiles sur la peau humaine et pour l'assujettissement de langes.
